# EUROPEAN PATENT SPECIFICATION

(11) **EP 3 027 046 B1**
(45) Date of publication and mention of the grant of the patent: **13.03.2019**
(21) Application number: 13890345.5
(22) Date of filing: 30.07.2013
(51) Int. Cl.: A23K 50/00

(54) **METHODS FOR FEEDING DIETARY COMPOSITIONS TO RUMINANTS**
VERFAHREN ZUM FÜTTERN VON WIEDERKÄUERN MIT NÄHRSTOFFZUSAMMENSETZUNGEN
PROCÉDÉS POUR FOURNIR DES COMPOSITIONS ALIMENTAIRES À DES RUMINANTS

(43) Date of publication of application: 08.06.2016
(73) Proprietor: Benemilk Oy, 21200 Raisio (FI)
(72) Inventor: ARONEN, Ilmo Pellervo, FI-27600 Hinnerjoki (FI); HOLMA, Merja Birgitta, FI-21200 Raisio (FI); WAN, Feng, Issaquah, Washington 98029 (US); BUNTEL, Christopher John, Singapore 276693 (SG)
(74) Representative: Gevers Patents
(86) International application number: PCT/US2013/052680
(87) International publication number: WO 2015/016833

(56) References cited:
- EP-A1- 1 707 059
- WO-A1-2008/013939
- US-A- 3 959 493
- US-A- 4 642 317
- US-A- 5 186 937
- US-A1- 2008 015 217
- US-A1- 2012 294 876
- S.A. MOSLEY ET AL: "Effect of Varying Levels of Fatty Acids from Palm Oil on Feed Intake and Milk Production in Holstein Cows", JOURNAL OF DAIRY SCIENCE, vol. 90, no. 2, 1 February 2007 (2007-02-01), pages 987-993, XP055187571, ISSN: 0022-0302, DOI: 10.3168/jds.S0022-0302(07)71583-7
- Anonymous: "March 2010 Newsletter", GP Feeds , March 2010 (2010-03), pages 1-6, XP002766596, Retrieved from the Internet: URL:http://www.gpfeeds.co.uk/newsletters/m ar10.htm [retrieved on 2017-01-24]
- DANN H M ET AL: "Diets During Far-Off and Close-Up Dry Periods Affect Periparturient Metabolism and Lactation in Multiparous Cows", JOURNAL OF DAIRY SCIENCE, vol. 89, no. 9, 1 September 2006 (2006-09-01), pages 3563-3577, XP026957198, AMERICAN DAIRY SCIENCE ASSOCIATION, US ISSN: 0022-0302 [retrieved on 2006-09-01]
- J.R. LOFTEN ET AL: "Invited review: Palmitic and stearic acid metabolism in lactating dairy cows", JOURNAL OF DAIRY SCIENCE., vol. 97, no. 8, 1 August 2014 (2014-08-01) , pages 4661-4674, XP055338108, US ISSN: 0022-0302, DOI: 10.3168/jds.2014-7919

## Description

### BACKGROUND

Increasing production and fat content of milk obtained from lactating ruminants has been a major goal for dairy farmers. Additional milk production per ruminant is beneficial because it results in a higher yield, thereby increasing profits. Increased milk fat is desirable because it has a higher economic value and can be used in highly desirable food products, such as cheese, yogurt, and the like.

Conventional approaches to increasing production yield and milk fat content includes adjusting feed, nutrients, elements, vitamins, supplements, and/or the like provided to the ruminant. However, the current methods and feeds used to increase milk fat content tend to lower milk production or vice versa, and do not adequately take the physical conditions and time periods associated with the ruminants into account. In addition, these methods and feeds may produce other detrimental effects, such as lower protein content, taste defects, preservation issues, and increased trans fatty acid levels. Accordingly, the milk production industry would benefit from a method of feeding ruminants that increases milk yield and milk fat content without producing negative consequences that may outweigh the benefits of the feed.

The article by S.A. Mosley et al. entitled "Effect of Varying Levels of Fatty Acids from Palm Oil on Feed Intake and Milk Production in Holstein Cows" (Journal of Dairy Science, Vol. 90, No. 2, 1 February 2007, pages 987 to 993, XP55187571, ISSN: 0022-0302, DOI: 10.3168j=/jds.S.022-0302(07)71583-7) discloses using fatty acids of palm oil to increase milk yield and milk fat percentage by feeding cows during midlactation periods.

### SUMMARY

In accordance with the present invention, a method for feeding a ruminant may include determining a body weight of the ruminant and a milk yield of the ruminant, and providing a ruminant feed product to the ruminant for ingestion according to a feed program is disclosed. The ruminant feed product may comprise at least one fatty acid component comprising at least 70% of a palmitic acid compound by weight. The feed program comprises feeding the ruminant feed product to the ruminant at a first level during a first time period, wherein the first level does not exceed 0.04 grams of the fatty acid component per kilogram of the body weight and the first time period comprises a far-off dry cow period in a range of six to three weeks before calving, feeding the ruminant feed product to the ruminant at a second level during a second time period, wherein the second level comprises at least 0.04 grams of the fatty acid component per kilogram of the body weight and the second time period comprises a close-up dry cow period in the range of three weeks before calving to calving, and feeding the ruminant feed product to the ruminant at a third level during a third time period, wherein the third level comprises at least 0.4 grams of the fatty acid component per kilogram of the body weight and the third time period is from calving to a far-off dry cow period.

In a non-claimed example, a method of feeding a ruminant may include determining a body weight of the ruminant and a milk yield of the ruminant, and providing a ruminant feed product to the ruminant for ingestion according to a feed program. The feed product may comprise at least one fatty acid component comprising at least about 70% of a palmitic acid compound by weight. The feed program may comprise feeding the ruminant feed product to the ruminant at a first level of at most about 0.04 grams of the fatty acid component per kilogram of the body weight during a first time period, wherein the first time period comprises a far-off dry cow period, feeding the ruminant feed product to the ruminant at a second level of at least 0.04 grams of the fatty acid component per kilogram of the body weight during a second time period, wherein the second time period comprises a close-up dry cow period , and feeding the ruminant feed product to the ruminant at a third level of at least 0.4 grams of fatty acid component per kilogram of the body weight during a third time period, wherein the third time period comprises at least one of a fresh cow period, a peak lactation period, a constant phase of lactation period, and a late lactation period.

In a non-claimed example, a system for feeding a ruminant may comprise a feed source configured to provide a ruminant feed product for ingestion by the ruminant, the ruminant feed product comprising at least one fatty acid component comprising at least about 70% of a palmitic acid compound by weight, and at least one feeding element having access to the feed source, the at least one feeding element being configured to feed the ruminant feed product to the ruminant according to a feed program. The feed program may comprise feeding the ruminant feed product to the ruminant at a first level during a first time period, wherein the first level does not exceed about 0.04 grams of the fatty acid component per kilogram of the body weight, feeding the ruminant feed product to the ruminant at a second level during a second time period, wherein the second level comprises at least 0.04 grams of the fatty acid component per kilogram of the body weight, and feeding the ruminant feed product to the ruminant at a third level during a third time period, wherein the third level comprises at least 0.4 grams of the fatty acid component per kilogram of the body weight, The invention is as defined by the appended claims.

### BRIEF DESCRIPTION OF THE DRAWINGS

FIG. 1 depicts a flow diagram of an illustrative method of feeding a ruminant according to a first embodiment.
FIG. 2 depicts a flow diagram of an illustrative method of feeding a ruminant according to a second embodiment.
FIG. 3 depicts a flow diagram of illustrative feed program according to a first embodiment.
FIG. 4 depicts a flow diagram of illustrative feed program according to a second embodiment.
FIG. 5 depicts an illustrative lactation cycle for a ruminant and a feed program for feeding the ruminant according to some embodiments.

### DETAILED DESCRIPTION

This disclosure is not limited to the particular systems, devices and methods described, as these may vary. The terminology used in the description is for the purpose of describing the particular versions or embodiments only, and is not intended to limit the scope.

As used in this document, the singular forms "a," "an," and "the" include plural references unless the context clearly dictates otherwise. Unless defined otherwise, all technical and scientific terms used herein have the same meanings as commonly understood by one of ordinary skill in the art. Nothing in this disclosure is to be construed as an admission that the embodiments described in this disclosure are not entitled to antedate such disclosure by virtue of prior invention. As used in this document, the term "comprising" means "including, but not limited to."

The following terms shall have, for the purposes of this application, the respective meanings set forth below.

A "ruminant" is generally a suborder of mammal with a multiple chamber stomach that gives the animal the ability to digest cellulose-based food by softening it within a first chamber (rumen) of the stomach and to regurgitate the semi-digested mass to be chewed again by the ruminant for digestion in one or more other chambers of the stomach. Examples of ruminants include, but are not limited to, lactating animals such as cattle, goats and sheep. Cattle may include dairy cows, which are generally animals of the species *Bos taurus.* The milk produced by ruminants is widely used in a variety of dairy-based products.

The present technology generally relates to methods for feeding dietary compositions to ruminants. The methods for feeding the dietary compositions may be configured to improve various aspects of milk production in the ruminants. For instance, some embodiments provide that the methods of feeding the dietary compositions may increase the amount of milk produced by the ruminant (for example, the milk yield) and/or increase the fat content of the milk produced by the ruminant. The dietary compositions may include ruminant feed product having a saturated fatty acid component that include at least about 70% of a palmitic acid compound by weight. The ruminant feed product may be configured to improve various aspects of milk production in the ruminants and may more considerably improve various aspects of milk production in the ruminants when fed according to feeding methods described herein.

The diet ingested by a ruminant may have different effects on the ruminant during different phases of its lifecycle and lactation cycle. Accordingly, methods for feeding ruminants provided according to some embodiments described herein may be configured to feed the ruminant feed product to the ruminant at various concentrations based on, among other things, various time periods associated with the ruminant. In an embodiment, the time periods may be based on the calving cycle of the ruminant. For instance, the ruminant may be fed certain concentrations of the ruminant feed product at different time periods before calving or different concentrations at different time periods after calving. However, embodiments are not limited to time periods based on the calving cycle as any time period capable of operating according to some embodiments is contemplated herein. For instance, time periods may include, without limitation, seasonal time periods, life stages (for example, juvenile, adult, or the like), health-based time periods (for example, the ruminant may be fed different concentrations of the ruminant feed product during times of good health as compared to times of illness), or the like. The concentrations may be determined based on, for example, the weight of the ruminant and/or the milk yield of the ruminant. In an embodiment, the milk yield may include kilograms of milk produced per day, per week, per year, or the like. For example, at a certain time period the ruminant may be fed an amount of ruminant feed product (for instance, grams) for every unit of weight of the ruminant (for example, kilograms) or every unit of milk production (for example, kilograms/day). In this manner, the milk yield and/or the fat content of the milk produced by the ruminant may be maximized based on certain time periods and characteristics (for example, body weight and milk yield) associated with the ruminant without encountering various deleterious effects associated with conventional feed and feeding methods.

The physical and/or dietary needs of a ruminant, such as a dairy cow, change as the cow progresses through the calving cycle, gives birth (calves), and enters the post-calving period. Increasing the fat content of the milk right after calving may cause a negative energy balance for the cow if not managed with proper feeding. The feed and feeding methods may have an effect on the quality of colostrum (for example, milk produced by a cow for a newborn calf) produced by the ruminant after calving. Colostrum is crucial for newborn calves because they do not experience the passive transfer of immunity via the placenta before birth, so any antibodies that they need have to be ingested. Increased amounts of high quality colostrum may decrease calf mortality and may allow the calves to grow faster and healthier. In addition, the high fat colostrum is a very important energy source for the calf because the body reserves of the calf are very limited. Accordingly, methods for feeding a ruminant may be configured to maximize fat content and milk yield while based on, among other things, the calving cycle of the ruminant.

When a ruminant consumes feed, the fat in the feed is modified by the rumen to provide a milk fat profile that is different from the profile of fat in the feed. All fats which are not completely inert in the rumen may decrease feed intake and rumen digestibility of the feed material. Milk composition and fat quality may be influenced by the ruminant's diet. For example, oil feeding (for instance, the feeding of vegetable oils) can have negative effects on both rumen function and milk formation. As a result of oil feeding, the milk protein concentration may be lowered, the fat concentration may be decreased, and the proportion of trans fatty acids may be increased. These results have been connected with various negative milk characteristics, such as an increase in the harmful low-density lipoprotein (LDL) cholesterol and a decrease in the beneficial high-density lipoprotein (HDL) cholesterol in human blood when the milk is consumed. In addition, the properties of the milk fat during industrial milk processing may be weakened. A high level of polyunsaturated fatty acids in milk can also cause taste defects and preservation problems. A typical fatty acid composition of milk fat may contain more than about 70% saturated fatty acids and a total amount of trans fatty acids may be from about 3% to about 10%. When vegetable oil is added into the feed, the proportion of trans fatty acids may rise to more than about 10%.

One solution to diminishing the detrimental effect of oil and fat is to prevent triglyceride fat hydrolysis. Fat hydrolysis can be decreased, for example, by protecting fats with formaldehyde treated casein. Another alternative is to feed the ruminant insoluble fatty acid calcium salts whereby hydrogenation in rumen can be avoided. However, fatty acid salts typically have a pungent taste that may result in decreased feed intake by the ruminant.

Accordingly, the ruminant feed product described herein may include a saturated fatty acid component that includes at least about 70% to about 100% of a palmitic acid compound by weight. In an embodiment, the saturated fatty acid component may include at least about 90% of the palmitic acid compound by weight. The dietary composition allows for the transfer of palmitic acid from the feed via the digestive tract into the blood circulation of a ruminant. This may improve the energy efficiency of milk production and the utilization of energy of the ruminant. When the utilization of energy becomes more effective, milk production may increase and/or the concentrations of protein and/or fat in the milk may rise. According to some embodiments, the dietary composition may be configured to enhance fat synthesis in the mammary gland by bringing milk fat components to the cell such that energy consuming synthesis in the mammary gland is not necessary. As a result, glucose may be used more efficiently for lactose production causing increased milk production. In addition, the milk protein content may increase because there is no need to produce glucose from amino acids. Thus, the ruminant therefore does not lose weight or loses less weight at the beginning of the lactation period, thereby improving the fertility of the ruminant.

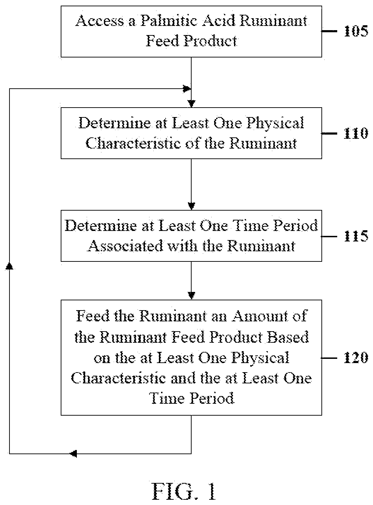

FIG. 1 depicts a flow diagram of an illustrative method for feeding a ruminant according to a first embodiment. In various examples, the components described herein with respect to FIG. 1 may generally be combined in any order and/or any combination, may include more or fewer components, and are not limited by the order described herein. In various examples, the feeding methods may be configured in a manner that maximizes particular qualities in the milk produced by the ruminant, as well as an amount of milk produced by the ruminant, as described in greater detail herein.

As depicted in FIG. 1, a palmitic acid ruminant feed product (the "ruminant feed product") may be accessed 105 for feeding to the ruminant. The ruminant feed product may be accessed 105 in various forms, such as a fluid, a dry or substantially dry material, pellets, loose particulates, or the like. According to some examples, the ruminant feed product may include at least one saturated fatty acid component containing at least about 70% of a palmitic acid compound by weight. The fatty acid component can generally be present in the ruminant feed product at any concentration. For example, some sample concentration ranges include about 5% by weight to about 80% by weight, about 10% by weight to about 70% by weight, and about 10% by weight to about 50% by weight. In some specific examples, the fatty acid component may be present in the ruminant feed product in an amount of about 5% by weight, about 10% by weight, about 15% by weight, about 20% by weight, about 25% by weight, about 30% by weight, about 40% by weight, about 50% by weight, or any value or range between any two of these values (including endpoints).

In some examples, the fatty acid component may have a melting point of at least about 40 °C, about 80 °C or less, or about 40 °C to about 80 °C. In particular examples, the fatty acid component may have a melting point of about 40 °C, about 45 °C, about 50 °C, about 55 °C, about 60 °C, about 65 °C, about 70 °C, about 75 °C, about 80 °C, or any value or range between any two of these values (including endpoints). The melting point may be selected so that it is a temperature that aids in keeping the fatty acid inert in the rumen environment.

The palmitic acid compound is not limited by this disclosure, and may include one or more of a conjugated palmitic acid, unconjugated palmitic acid, free palmitic acid, palmitic acid derivatives, and/or the like. Palmitic acid, also known as hexadecanoic acid, has a molecular formula of CH₃(CH₂)₁₄CO₂H. Non-limiting examples of palmitic acid derivatives include palmitic acid esters, palmitic acid amides, palmitic acid salts, palmitic acid carbonates, palmitic acid carbamates, palmitic acid imides, palmitic acid anhydrides, and/or the like. According to some examples, the palmitic acid compound may include free palmitic acid, palmitate triglyceride, sodium palmitate, calcium palmitate, magnesium palmitate, ammonium palmitate, or combinations thereof.

The palmitic acid compound may be present in the saturated fatty acid component in an amount of about 60% by weight of the fatty acid to about 100% by weight of the fatty acid, including about 60% by weight, about 65% by weight, about 70% by weight, about 75% by weight, about 80% by weight, about 85% by weight, about 90% by weight, about 95% by weight, about 98% by weight, about 99% by weight, about 100% by weight, or any value or range between any two of these values (including endpoints). In an example, the saturated fatty acid component may consist of 100% of the palmitic acid compound, in other words, the saturated fatty acid component is palmitic acid.

In some examples, the saturated fatty acid component may include a stearic acid compound. The stearic acid compound is not limited by this disclosure, and may include conjugated stearic acid, unconjugated stearic acid, free stearic acid, stearic acid derivatives, and/or the like. Stearic acid, also known as octadecanoic acid, has a molecular formula of CH₃(CH₂)₁₆CO₂H. Specific examples of stearic acid derivatives may include stearic acid esters, stearic acid amides, stearic acid salts, stearic acid carbonates, stearic acid carbamates, stearic acid imides, stearic acid anhydrides, and/or the like. Because stearic acid in large amounts may hinder milk production capacity of the mammary gland, the amount of stearic acid may be present in the fatty acid component in an amount of about 30% or less by weight of the fatty acid component. In particular examples, the stearic acid compound may include about 30% by weight of the fatty acid component, about 25% by weight of the fatty acid component, about 20% by weight of the fatty acid component, about 15% by weight of the fatty acid component, about 10% by weight of the fatty acid component, about 5% by weight of the fatty acid component, about 1% by weight of the fatty acid component, about 0% by weight of the fatty acid component, or any value or range between any two of these values. In some examples, the fatty acid component substantially does not contain a stearic acid compound.

According to some embodiments, the saturated fatty acid component may be free or substantially free of trans fatty acids. For example, a trans fatty acid component may be present in the ruminant feed product in an amount of about 5% by weight, about 3% by weight, about 2% by weight, about 1% by weight, about 0.5% by weight, about 0% by weight, or any value or range between any two of these values (including endpoints).

The ruminant feed product accessed **105** according to the method depicted in FIG. 1 may include other components, for instance, one or more nutrient components. The nutrient component may include, without limitation, carnitine, at least one glucogenic precursor, at least one vitamin, at least one mineral, at least one amino acid, at least one amino acid derivative, at least one antioxidant, at least one protein, or combinations thereof. The ruminant feed product may include various portions of the one or more nutrient components generally included in particular amounts that are sufficient to provide beneficial nutritional and dietary needs of the ruminant that is to consume the dietary composition. For example, the ruminant feed product may include a carbohydrate portion and a vitamin portion, each in an amount sufficient to provide beneficial nutritional and dietary needs of the ruminant.

In some examples, the feed ingredient may include an amount of carnitine. The carnitine may be included in the feed ingredient to aid in the breakdown of fatty acids to generate metabolic energy in the ruminant. In some examples, the carnitine may be present in a premix composition.

In various examples, the glucogenic precursor may include at least one of glycerol, propylene glycol, molasses, propionate, glycerine, propane diol, calcium propionate, propionic acid, octanoic acid, steam-exploded sawdust, steam-exploded wood chips, steam-exploded wheat straw, algae, algae meal, microalgae, or combinations thereof. The glucogenic precursor may generally be included in the feed ingredient to provide an energy source to the ruminant, for example, so as to prevent gluconeogenesis from occurring within the ruminant' s body.

The antioxidant is not limited by this disclosure and may include any antioxidants or combination of antioxidants, particularly those used in the ruminant feed product. Illustrative examples of antioxidants may include alpha-carotene, beta-carotene, ethoxyquin, butylated hydroxyanisole (BHA), butylated hydroxytoluene (BHT), cryptoxanthin, lutein, lycopene, zeaxanthin, vitamin A, vitamin C, vitamin E, selenium, alpha-lipoic acid, and/or the like.

In various examples, the vitamin may include any combination of vitamins including, without limitation, vitamin A, vitamin B, vitamin D, vitamin E, vitamin C, vitamin K, and/or the like. Specific examples of vitamin B include thiamine (vitamin B₁), riboflavin (vitamin B₂), niacin (vitamin B₃), pantothenic acid (vitamin B₅), pyridoxine (vitamin B₆), biotin (vitamin B₇), folic acid (vitamin B₉), cobalamin (vitamin B₁₂), and choline (vitamin Bₚ)_{.}

In various examples, the amino acid may include any combination of common, uncommon, essential, and non-essential amino acids, including, without limitation, essential amino acids such as leucine, lysine, histidine, valine, arginine, threonine, isoleucine, phenylalanine, methionine, tryptophan, and/or any derivative thereof. In some embodiments, the amino acid may be a non-essential amino acid, including any combination of alanine, asparagine, aspartate, cysteine, glutamate, glutamine, glycine, proline, serine, tyrosine, and/or any derivative thereof. The amino acid and/or any derivative thereof may also include amino acids and derivatives of both non-essential and essential amino acids. The amino acid may generally be included in the feed ingredient to provide a nutritional aid in various physiological processes in the ruminant, such as, for example, increasing muscle mass, providing energy, aiding in recovery, and/or the like. In some examples, the amino acid may be present in a premix composition.

In various examples, the mineral may be any mineral that is a generally recognized as safe (GRAS) mineral or a combination of such minerals. The mineral may further be obtained from any mineral source that provides a bioavailable mineral. In some examples, the mineral may be one or more of calcium, sodium, magnesium, potassium, phosphorous, zinc, selenium, manganese, iron, cobalt, copper, iodine, molybdenum, and/or the like. In some examples, the mineral may be selected from one or more of a sodium salt, a calcium salt, a magnesium salt, a cobalt salt, a manganese salt, a potassium salt, an iron salt, a zinc salt, copper sulfate, copper oxide, selenium yeast, a chelated mineral, and/or the like. Illustrative examples of sodium salts include monosodium phosphate, sodium acetate, sodium chloride, sodium bicarbonate, disodium phosphate, sodium iodate, sodium iodide, sodium tripolyphosphate, sodium sulfate, sodium selenite, and/or the like. Illustrative examples of calcium salts include calcium acetate, calcium carbonate, calcium chloride, calcium gluconate, calcium hydroxide, calcium iodate, calcium iodobehenate, calcium oxide, anhydrous calcium sulfate, calcium sulfate dehydrate, dicalcium phosphate, monocalcium phosphate, tricalcium phosphate, and/or the like. Illustrative magnesium salts include magnesium acetate, magnesium carbonate, magnesium oxide, magnesium sulfate, and/or the like. Illustrative cobalt salts include cobalt acetate, cobalt carbonate, cobalt chloride, cobalt oxide, cobalt sulfate, and/or the like. Illustrative examples of manganese salts include manganese carbonate, manganese chloride, manganese citrate, manganese gluconate, manganese orthophosphate, manganese oxide, manganese phosphate, manganese sulfate, and/or the like. Illustrative examples of potassium salts include potassium acetate, potassium bicarbonate, potassium carbonate, potassium chloride, potassium iodate, potassium iodide, potassium sulfate, and/or the like. Illustrative examples of iron salts include iron ammonium citrate, iron carbonate, iron chloride, iron gluconate, iron oxide, iron phosphate, iron pyrophosphate, iron sulfate, reduced iron, and/or the like. Illustrative examples of zinc salts include zinc acetate, zinc carbonate, zinc chloride, zinc oxide, zinc sulfate, and/or the like.

In some examples, the protein used in the feed ingredient may be obtained from a protein source. Illustrative examples of protein sources may include one or more grains and/or oilseed meals. The grain is generally not limited by this disclosure and may be any edible grain, or combination of grains, that is used as a protein source. Illustrative examples of grains include cereal grains such as barley, millet, wheat, spelt wheat, rye, oats, triticale, rice, corn, buck wheat, quinoa, amaranthus, sorghum, and the like. Oilseed meal is generally derived from residue that remains after reserved oil is removed from oilseeds. The oilseed meal may be rich in protein and variable in residual fats and oils. Illustrative examples of oilseed meal includes rapeseed meal, soybean meal, sunflower meal, cottonseed meal, camelina meal, mustard seed meal, crambe seed meal, safflower meal, rice meal, peanut meal, corn gluten meal, corn gluten feed, distillers dried grains, distillers dried grains with solubles, wheat gluten, and/or the like. According to some embodiments, the ruminant feed product may include materials such as algae, algae meal, microalgae, or the like.

In some examples, the ruminant feed product may include at least one cellulosic material. The cellulosic material may generally provide a source of fiber for the ruminant to lower cholesterol levels and promote proper digestive function. Illustrative examples of cellulosic materials include wheat bran, wheat middlings, wheat mill run, oat hulls, oat bran, soya hulls, grass meal, hay meal, alfalfa meal, alfalfa, straw, hay, and/or the like.

In various examples, the ruminant feed product may include a micronutrient mixture. Micronutrient mixtures are not limited by this disclosure and may generally contain any micronutrient mixture now known or later developed. The micronutrient mixture may include various components, such as at least one vitamin and at least one mineral, as described in greater detail herein. In some examples, the micronutrient mixture may be present in a premix composition.

In various examples, the ruminant feed product or portions thereof may be subjected to a grinding process configured to form the feed ingredient or portions thereof into certain particle sizes and/or to achieve a more uniform particle size. For example, a carbohydrate and/or a protein component of the feed ingredient may be ground to a certain particle size. In another example, the feed ingredient itself may be ground to a certain particle size.

Grinding may be performed by various grinding devices known to those having ordinary skill in the art, such as a hammer mill, a roller mill, a disk mill, or the like. The feed ingredient and/or portions thereof may be ground to various sizes, such as particle size (for instance, measured in millimeters), mesh sizes, surface areas, or the like. According to some examples, the feed ingredient and/or portions thereof may be ground to a particle size of about 0.05 millimeters, about 1 millimeters, about 2 millimeters, about 5 millimeters, about 7 millimeters, about 10 millimeters, and values or ranges between any two of these values (including endpoints). In some examples, the various components may be ground so that about 20% to 50% of the each component and/or all components are retained by a mesh having openings with a size of about 10 mm and so that about 70% to about 90% of each component and/or all components are retained by a mesh having openings with a size of about 1 mm. In some examples, the various components may have a varying distribution of particle sizes based upon the ingredients. For example, in examples containing one or more wheat ingredients, the particle size may be distributed so that about 95% of the ground wheat ingredients are retained by a mesh having openings with a size of about 0.0625 mm and so that about 65% of the ground wheat ingredients are retained by a mesh having openings with a size of about 1.0 mm. In another example, such as examples containing one or more barley ingredients, the particle size may be distributed so that about 95% of the ground barley ingredients are retained by a mesh having openings with a size of about 0.0625 mm and so that about 60% of the ground barley ingredients are retained by a mesh having openings with a size of about 1.0 mm. The varying mesh sizes of each ingredient may be independent of mesh sizes for other ingredients.

Grinding may provide various benefits, such as improving certain characteristics of the feed ingredient and/or the dietary composition formed therefrom. For instance, even and fine particle size may improve the mixing of different ingredients. According to certain examples, grinding may be configured to decrease a particle size of certain components of the dietary composition, for example, to increase the surface area open for enzymes in the gastrointestinal tract, which may improve the digestibility of nutrients, and/or to increase the palatability of the feed.

In an example, the ruminant feed product may include water. For example, the water content of the ruminant feed product may be about 5% by weight, about 10% by weight, about 12% by weight, about 15% by weight, about 25% by weight or any value or range between any two of these values (including endpoints). In an example, the ruminant feed product may include a carbohydrate component and/or a nitrogen component. The carbohydrate component may generally include at least one of a sugar, a starch, or a grain. Non-limiting examples of carbohydrate components include cellulose, hemicellulose, sugar beet pulp, sugar cane, wheat bran, oat hulls, grain hulls, soybean hulls, peanut hulls, wood, brewery by-product, algae, algae meal, grasses, legumes, plant-based feedstuffs, wheat, corn, oats, sorghum, millet, and barley. Non-limiting examples of the nitrogen component include oilseed meals, soy meals, bean meals, rapeseed meals, sunflower meals, coconut meals, olive meals, linseed meals, and grapeseed meals. The ruminant feed product may include various concentrations of the carbohydrate component and/or the nitrogen component. For example, the carbohydrate component content of the ruminant feed product may be about 0.1% to about 55% by weight and the nitrogen component of the ruminant feed product may be about 0.1% to about 55% by weight. In an embodiment, the ruminant feed product may include a micellizing agent. Non-limiting examples of micellizing agents may include lecithin, cephalin, castor oil ethoxylate, sorbitan monooleate, tallow ethoxylate, lauric acid, polyethylene glycol, or combinations thereof.

Referring to FIG. 1, at least one physical characteristic of the ruminant may be determined 110. The at least one physical characteristic may include, without limitation, the age, weight, pedigree or heritage, genetics, physical condition, health condition, milk yield, milk fat content, or the like. According to some examples, the at least one physical characteristic may be determined 110 at various times, such as periodically (for instance, daily, weekly, monthly, or the like) or according to various triggers, such as a noticeable change in a characteristic of the ruminant (for instance, an illness, a calving event, or the like). At least one time period associated with the ruminant may be determined 115. The at least one time period may include, without limitation, age, calving cycle, life stage, illness progression, season, lactation cycle, or the like. The at least one physical characteristic of the ruminant and the at least one time period associated with the ruminant may be determined **110, 115** and used as an indicator of the physical condition and/or the dietary needs of the ruminant.

Lactation cycles have various "periods" defined relative to the calving event. For example, in dairy cows, the "dry cow period" is the period from about eight or six weeks before calving until calving. In general, milk product may begin at calving and may end by at or just before the dry period. The dry cow period is subdivided into the "far-off dry cow period", which is from about eight or six weeks before calving to three weeks before calving, and the "close-up dry cow period", which is from about three weeks before calving until calving. The period from calving until about three weeks after calving is called the "fresh cow period". Lactation (milk production) occurs during and after the fresh cow period. "Peak lactation" typically occurs sometime around six to nine weeks after calving. After peak lactation, there is a "constant phase" of relatively constant milk production, followed by "late lactation" during which milk production gradually decreases.

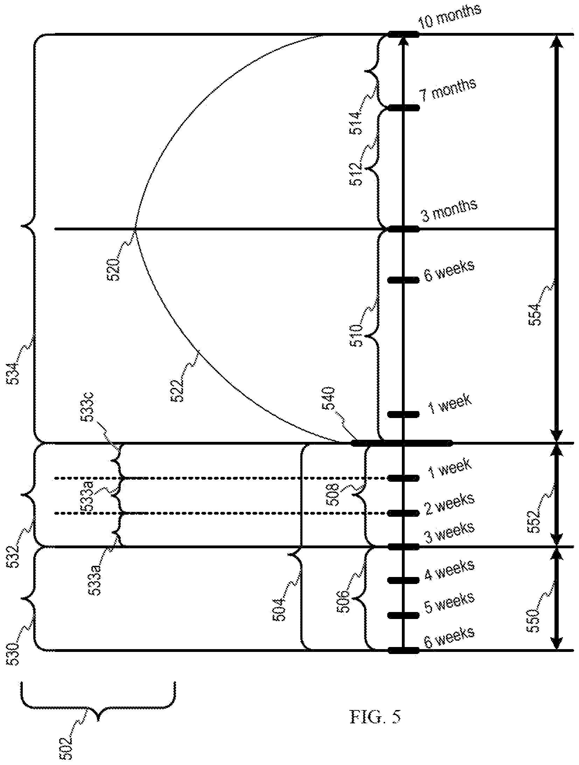

Ruminants, such as dairy cows, typically eat more food after calving than before calving. For example, in the dry cow period, a dairy cow might eat about 1.5% to about 2.5% by weight of dry matter. For example, the dairy cow might eat about 2% of their body weight in dry matter per day. After calving, the dairy cow gradually increases food intake until the cow is eating about 4% to about 7% by weight of dry matter. For example, the cow might eat about 5% of their body weight in dry matter per day. Dry matter includes any solid state feed materials that the cow ingests including, for example, pellets feed, loose particulate feed, or total mixed ration (TMR) feed. In general, TMR is a mix of grain and silage with some protein meals, such as, for example, soya bean meal and canola meal. Additional materials and trace elements, vitamins, extra nutrients, and the like may also be added to the TMR. Refer to FIG. 5 for an example illustration of a lactation cycle and a feed program for a ruminant during various phases of the lactation cycle according to some embodiments described herein.

In some general examples, various periods or sub-periods within the lactation cycle can be used to vary the amount of a ruminant feed product or the amount of saturated fatty acid fed to the ruminant. For example, a first level C1 can be fed to the ruminant during a first time period PI, a second level C2 can be fed to the ruminant during a second time period P2, and a third level C3 can be fed to the ruminant during a third time period P3. The second level C2 may be higher than the first level C1. The level may be the concentration or amount. The level may gradually increase from C1 to C2, or may increase in one or more steps.

In some examples, the first time period P1 may include the far-off dry cow period. For example, P1 can be from about eight to six weeks before calving to about calving. In one example, P1 can be from about eight weeks before calving to about calving. In another example, P1 can be from about six weeks before calving to about calving. In some examples, C1, as the concentration or amount of the grams of the palmitic acid compound per kilogram of body weight ("grams/kilogram" or "g/kg"), can be at most 0.04 grams/kilogram. In some examples, C1 can be less than 0.02 grams/kilogram. In some examples, substantially no or no palmitic acid compound is fed to the ruminant during P1.

In some examples, the second time period P2 may include the close-up dry cow period. For example P2 can be about three weeks before calving to about calving.

The second time period P2 can be further divided into multiple sub-periods such as P2a, P2b, and P2c. For example, when P2 is the three-week long period before calving, sub-period P2a can be about three weeks before calving to about two weeks before calving, P2b can be about two weeks before calving to about one week before calving, and P2c can be about one week before calving to calving (for instance, less than a week before calving to about less than a week after calving). Concentration or amount C2a can be fed to the ruminant during P2a, concentration or amount C2b can be fed to the ruminant during P2b, and concentration or amount C2c can be fed to the ruminant during P2c. Concentration or amount can be the grams of a saturated fatty acid (such as palmitic acid) per kilogram of body weight ("grams/kilogram" or "g/kg"). For example, C2a can be about 0.04 grams/kilogram to about 0.08 grams/kilogram body weight. For example, C2b can be about 0.08 grams/kilogram to about 0.16 grams/kilogram body weight. For example, C2c can be about 0.14 grams/kilogram to about 0.22 grams/kilogram body weight.

In some examples, the third time period P3 may include at least one of the fresh cow period, the peak lactation period, the constant phase of lactation period, and the late lactation phase, or any combinations. For example, P3 can be from calving to about 48 weeks after calving. In yet other examples, P3 can include all or a portion of the fresh cow period, the peak lactation period, the constant phase of lactation period, and the late lactation phase. For example, P3 can be from about three days after calving to about 48 weeks after calving. Alternatively, P3 can be from about calving to about nine weeks after calving. Alternatively, P3 can be about one week after calving to about 24 weeks after calving. Alternatively, P3 can be about one week after calving to about 36 weeks after calving. Alternatively, P3 can be about one week after calving to about 42 weeks after calving. In some examples, concentration or amount C3 is greater than C2. In some examples, when the concentration or amount is the grams of a saturated fatty acid (such as palmitic acid) per kg of body weight, C3 may be about 2, about 3, about 4, about 5, about 6, about 7, about 8, about 9, or about 10 times C2.

In some examples, the time period P3 can be after the fresh cow period. For example, P3 can be about three weeks or more after calving until the far-off dry cow period. In some examples, concentration or amount C3 is defined by grams of palmitic acid compound per kilogram of milk yield. For example, C3 can be about 5 grams/kilograms, about 7.5 grams/kilograms, about 10 grams/kilograms, about 12.5 grams/kilograms, about 15 grams/kilograms, or ranges between any two of these values (including endpoints).

The ruminant may be fed **120** an amount of the ruminant feed product based on the at least one physical characteristic and the at least one time period. For example, the ruminant may be fed a certain amount of the ruminant feed product, the saturated fatty acid compound, or the palmitic acid compound based on the weight and the age of the ruminant, such as X grams of the ruminant feed product for every Y kilograms of weight of the ruminant between age T and age U, 2X grams of the ruminant feed product for every Y kilograms of weight of the ruminant between age V and age W, and so on. In another example, the amount of the ruminant feed product, the saturated fatty acid compound, or the palmitic acid compound may be increased or decreased while the ruminant is experiencing an illness or during certain seasons (for instance, the ruminants may require more energy during certain seasons as compared to other seasons). Feeding the ruminant amounts of the ruminant feed product based on the physical and/or dietary needs of the ruminant as determined by a time period associated with the ruminant may improve the milk yield and/or fat content of the milk. The physical and/or dietary needs may include, without limitation, energy needs, growth needs, milk production needs, or the like.

According to some examples, different physical characteristics of the ruminant may be more indicative of the amount of the ruminant feed product that may be fed to the ruminant to improve milk yield and/or fat content at different time periods associated with the ruminant. As such, the amount of the ruminant feed product fed 120 to the ruminant may be based on different physical characteristics at different time periods. For example, the weight of the ruminant may be used during a first time period, the age of the ruminant may be used during a second time period, the milk yield of the ruminant may be used during a third time period, and so on. According to some examples, the diet of the ruminant may consist or consist essentially of only the ruminant feed product with the exception of, for example, medicine, supplements, or the like.

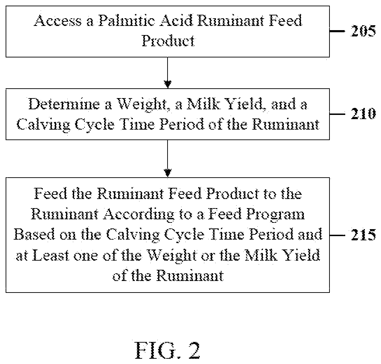

FIG. 2 depicts a method for feeding a ruminant according to a second embodiment. As shown in FIG. 2, a palmitic acid ruminant feed product may be accessed **205** for feeding to the ruminant. Characteristics of the ruminant including the weight, milk yield and calving cycle time period associated with the ruminant may be determined **210.** According to some examples, the calving cycle time period may be configured as a certain amount of time before or after the ruminant is scheduled to calve or has calved. Non-limiting examples of calving cycle time periods include 4 weeks before calving, 3 weeks before calving, 2 weeks before calving, 1 week before calving, 1 week after calving, 2 weeks after calving, 3 weeks after calving, 4 weeks after calving, and a non-calving period (for instance, 5 or more weeks after calving), and values or ranges between any two of these values (including endpoints). As described herein, the calving cycle time periods may be described in terms of time periods, including, without limitation, the dry cow period, the far- off dry cow period, the close-up dry cow period, the fresh cow period, peak lactation, constant phase, late lactation, or the like.

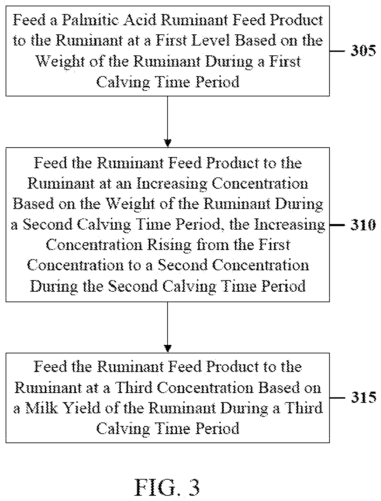

The calving cycle time period may be used to indicate the physical and/or dietary needs of the ruminant. The ruminant may be fed **215** according to a feed program configured based on the calving cycle time period and at least one of the weight or the milk yield of the ruminant (see FIG. 3, below, for an illustrative feed program according to some embodiments). The feed program may be used to feed **215** the ruminant in a manner that improves the milk yield and/or milk fat content of milk, including the colostrum, produced by the ruminant in view of the physical and/or dietary needs of the ruminant as the ruminant progresses through the calving cycle.

According to some examples, a system, such as an automatic cattle feeding system, may be used to perform various steps of the methods described herein. The system may include various components, including, without limitation, a ruminant feed product source, elements for accessing the ruminant feed product from the ruminant feed product source, ruminant feed product containers (for example, troughs, lick tanks, individual feed containers, or the like), and elements for measuring and/or dispensing the ruminant feed product. In an example, the system may include ruminant identification devices configured to identify at least one ruminant and to adjust a feed program to correspond with the at least one ruminant. For example, the system may be configured to dispense an amount of the ruminant feed product to a particular ruminant based on the physical characteristics of the ruminant, a time period associated with the ruminant, and the concentration of the ruminant feed product based on the physical characteristics and the time period.

FIG. 3 depicts an illustrative feed program according to a first embodiment. As shown in FIG. 3, a feed product may be fed **305** to the ruminant at a first concentration based on the weight of the ruminant during a first calving time period (or "first time period"). The feed product may or may not include palmitic acid. In various examples, the first calving time period may include from about 9 weeks, 8 weeks, 7 weeks, or 6 weeks before calving to about 3 weeks before calving. The palmitic acid ruminant feed product may include a feed product including a fatty acid component, such as a palmitic acid component. Accordingly, the amount, concentration, level or other measure of the fatty acid component may be adjusted as described herein to provide the ruminant with a concentration, level, amount, or the like of the fatty acid component. The remainder of the ruminant feed product, for instance, the portion of the ruminant feed product to be fed to the ruminant that is not the fatty acid component, may include dry matter and other materials described herein. According to some examples, the first level may be less than 0.04 grams of palmitic acid per kilogram of body weight ("grams/kilograms" or "g/kg"). According to some examples, the first level may be substantially free of palmitic acid.

The ruminant may be fed **310** the fatty acid component at an increasing concentration based on the weight of the ruminant during a second calving time period (or "second time period"). In various examples, the second calving time period may be from about 3 weeks before calving, about 2 weeks before calving, about 1 week before calving, to calving. According to some examples, the second level may include about 0.04 grams of palmitic acid per kilogram of body weight ("grams/kilograms"), about 0.08 grams of palmitic acid per kilogram of body weight, about 0.16 grams of palmitic acid per kilogram of body weight, about 0.22 grams of palmitic acid per kilogram of body weight, or values or ranges between any two of these values (including endpoints). For instance, for a ruminant having a body weight of about 750 kilograms, the amount of palmitic acid fed to the ruminant may be about 60 grams, about 127.5 grams, about 255 grams, about 375 grams, or values or ranges between any two of these values (including endpoints). In an embodiment, the second level may be determined based on feeding a certain number of grams (for instance, about 50 grams to about 100 grams) of palmitic acid or the fatty acid component per 600 kilograms of body weight of the ruminant.

The palmitic acid ruminant feed product may be fed **315** to the ruminant at a third level during a third calving time period (or "third time period"). The third level may be based on palmitic acid grams per kilogram of body weight or palmitic acid grams per kilogram of milk yield by the ruminant. In various examples, the third calving time period may include from calving to about 1 week after calving, about 2 weeks after calving, about 3 week after calving, about 5 weeks, about 9 weeks, about 20 weeks, about 40 weeks or more after calving, or values or ranges between any two of these values (including endpoints). According to some examples, the third level may be at least 0.4 gram palmitic acid per kilogram of body weight from about calving to about 1 week after calving. According to some examples, the third level may include about 2 grams of the fatty acid component per kilogram of milk yield, about 5 grams of the fatty acid component per kilogram of milk yield, about 10 grams of the fatty acid component per kilogram of milk yield, about 20 grams of ruminant feed product per kilogram of milk yield, or values or ranges between any two of these values (including endpoints), from one week after calving to until the far-off dry cow period. The third calving time period may include all or a portion of post-calving time period for the ruminant up to the far-off dry cow period. In an embodiment, feeding **315** the palmitic acid ruminant feed product to the ruminant at an increasing concentration based on the weight of the ruminant during the third calving time period may allow the ruminant to produce about 7 kilograms to about 10 kilograms of colostrum in a first milking after calving.

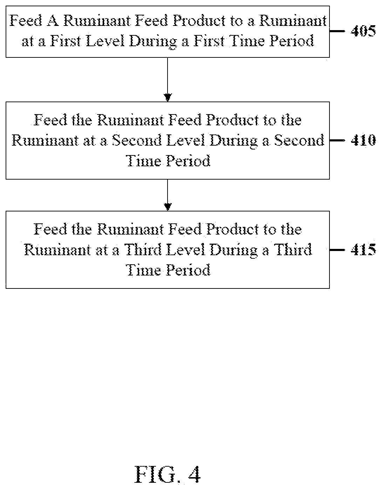

FIG. 4 depicts an illustrative feed program. As shown in FIG. 4, a ruminant feed product may be fed **405** to a ruminant at a first level during a first time period. According to some examples, the first level include a particular amount or concentration of the fatty acid component and/or ruminant feed dry matter of the ruminant feed product. In an example, the first level may be free or substantially free of the saturated fatty acid component. In various examples, the fatty acid component in the first level may not exceed about 0.01 grams per kilogram of the body weight of the ruminant ("grams/kilogram"), about 0.02 grams/kilogram, about 0.04 grams/kilogram, about 0.06 grams/kilogram, and ranges and values between any two of these values (including endpoints). In another example, the first level may include ruminant dry matter at about 1.0% of the body weight of the ruminant, about 1.5% of the body weight of the ruminant, about 2.0 % of the body weight of the ruminant, about 2.5% of the body weight of the ruminant, and ranges and values between any two of these values (including endpoints).

In an example, the first time period may include a dry cow period, such as a far-off dry cow period. In various examples, the first time period may include about 9 weeks, about 8 weeks, about 7 weeks, about 6 weeks, about 5 weeks, about 4 weeks, or about 3 weeks before calving to about calving.

The ruminant feed product may be fed **410** to the ruminant at a second level during a second time period. In an embodiment, the fatty acid component in the second level may include at least about 0.04 grams/kilogram, about 0.06 grams/kilogram, about 0.08 grams/kilogram, about 0.10 grams/kilogram, about 0.20 grams/kilogram, about 0.30 grams/kilogram, about 0.40 grams/kilogram and ranges and values between any two of these values (including endpoints). In an example, the second time period may include a dry cow period, such as a close-up dry cow period. In various examples, the second time period may include about 4 weeks before calving, about 3 weeks before calving, about 2 weeks before calving, about 1 week before calving, a calving time period (for instance, less than a week before calving to about less than a week after calving), and ranges and values between any two of these values (including endpoints).

In an example, the second level may include various concentrations of the ruminant feed product and/or components thereof during various sub-time periods of the second time period. For example, the second level may include a first concentration during a first sub-time period, a second concentration during a second sub-time period, a third concentration during a third sub-time period, and so on. In an example, the first sub-time period may include about 3 weeks before calving, the second sub-time period may include about 2 weeks before calving, and the third sub-time period may include about 1 week before calving.

In various examples, the first concentration may be about 0.01 gram/kilogram, about 0.02 grams/kilogram, about 0.04 grams/kilogram, about 0.05 grams/kilogram, about 0.06 grams/kilogram, about 0.08 grams/kilogram, about 0.10 grams/kilogram, and ranges and values between any two of these values (including endpoints). In various examples, the second concentration may be about 0.02 gram/kilogram, about 0.04 grams/kilogram, about 0.06 grams/kilogram, about 0.08 grams/kilogram, about 0.10 grams/kilogram, about 0.12 grams/kilogram, about 0.14 grams/kilogram, about 0.16 grams/kilogram, about 0.20 grams/kilogram and ranges and values between any two of these values (including endpoints). In various examples, the third concentration may be about 0.02 gram/kilogram, about 0.04 grams/kilogram, about 0.08 grams/kilogram, about 0.10 grams/kilogram, about 0.14 grams/kilogram, about 0.18 grams/kilogram, about 0.20 grams/kilogram, about 0.22 grams/kilogram, about 0.26 grams/kilogram and ranges and values between any two of these values (including endpoints).

A ruminant feed product may be fed **415** to the ruminant at a third level during a third time period. In various examples, the fatty acid component in the third level may include at least about 0.4 grams/kilogram, about 0.5 grams/kilogram, about 0.6 grams/kilogram, about 0.8 grams/kilogram, about 1.0 grams/kilogram, about 1.2 grams/kilogram, about 1.5 grams/kilogram, and ranges and values between any two of these values (including endpoints). In an example, the fatty acid component in the third level may include at most about 0.5 grams/kilogram. In another example, the third level may include ruminant dry matter at about 2% of the body weight of the ruminant, about 4% of the body weight of the ruminant, about 6% of the body weight of the ruminant, about 7% of the body weight of the ruminant, about 10% of the body weight of the ruminant, and ranges and values between any two of these values (including endpoints).

According to some examples, the third time period may include various lactation periods, such as a fresh cow period, a peak lactation period, a constant phase of lactation period, and a late lactation period. In various examples, the third time period may include a calving time period (for instance, less than a week before calving to about less than a week after calving) to a far-off dry cow period.

The third level of the ruminant feed product fed **415** may vary according to the particular third time period. In an example, the third level may include one of the following during the first week after calving: about 0.4 grams to about 0.8 grams/kilogram; at most 0.5 grams/kilogram during the first week after calving; about 10 grams of the fatty acid component per kilogram of the milk yield. In an example, the third level may include at least 0.4 grams/kilogram during the fresh cow period. In various examples, the third level may be at least about 8 grams of the fatty acid component per kilogram of the milk yield, about 10 grams of the fatty acid component per kilogram of the milk yield, about 12 grams of the fatty acid component per kilogram of the milk yield, about 16 grams of the fatty acid component per kilogram of the milk yield, and ranges and values between any two of these values (including endpoints) during the peak lactation period, the constant phase of lactation, and/or the late lactation period.

FIG. 5 depicts an illustrative lactation cycle for a ruminant (for instance, a dairy cow) and method for feeding the ruminant according to a feed program configured according to some embodiments. As shown in FIG. 5, the lactation cycle of a dairy cow may include various time periods that may be measured relative to a calving **540** event. For example, a dry cow period **504** may extend from about 8 or 6 weeks before calving **540** to calving. The dry cow period 504 may include a far-off dry cow period **506,** about 8 or 6 weeks before calving **540** to about 3 weeks before calving, and a close-up dry cow period 508, about 3 weeks before calving to about calving. A fresh cow period **510** may extend from about calving **540** to about 8 weeks after calving. A constant phase of lactation period **512** may extend for about 3 weeks to about 4 weeks from the end of the fresh cow period **510.** A 100-day late lactation period **514** may extend up until the dry cow period **504** for the ruminant. The dry cow period **504** may follow the 100-day late lactation period **514** as the lactation cycle repeats for the ruminant (for instance, if the ruminant calves again, such as once a year (about 350 days to about 425 days)). A lactation curve **520** graphically represents the lactation cycle for a ruminant over the various lactation periods **504-514,** including a peak lactation **522** that may generally occur between the end of the fresh cow period 510 and the beginning of the constant phase of lactation **512.**

According to some examples, the various lactation periods **504-514** may be segmented into time periods **550-554** for feeding the dairy cow at a particular level **530-534** during the lactation cycle. For example, a first time period **550** may extend through the far-off dry cow period **506** and may include feeding the dairy cow at a first level **530,** which may be about 0 grams of a fatty acid component per kilogram of body weight of the dairy cow ("grams/kilograms") to at most 0.4 grams/kilogram. As shown in FIG. 5, the fatty acid component may include a palmitic acid component of a ruminant feed product. A second time period **552** may extend through the close-up dry cow period 508 and may include feeding the dairy cow at a second level **532** of about 0.04 grams/kilogram to about 0.12 grams/kilogram.

The second level **532** may be segmented into multiple concentrations **533a-533c.** The concentrations may include a first concentration 533a of about 0.04 grams/kilogram to about 0.08 grams/kilogram, a second concentration **533a** of about 0.08 grams/kilogram to about 0.16 grams/kilogram, and a third concentration **533a** of about 0.14 grams/kilogram to about 0.22 grams/kilogram.

A third time period **554** may start after calving **540** and may include a third level **534** in which the dairy cow is fed about 0.4 grams/kilogram to about 0.5 grams/kilogram from calving to about one week after calving, then 10 grams of the fatty acid component per kilogram of milk for the dairy cow.

In various examples, methods for increasing milk fat content in milk produced by ruminants may include providing a ruminant feed product as described herein to the ruminant for ingestion. In some examples, providing the dietary composition to the ruminant for the ruminant to consume may result in an increase in production of milk and/or an increase in fat content of the milk produced. These increases may generally be relative to a similar ruminant that does not receive the dietary composition according to feed methods described herein, an average of similar ruminants not receiving the dietary composition according to feed methods described herein, an average of the milk production quantity and fat content of the same ruminant when not provided the dietary composition according to feed methods described herein, and/or the like. In particular examples, the milk production (or milk yield) may increase by an amount of about 1% to about 10%, including about 1%, about 2%, about 3%, about 4%, about 5%, about 6%, about 7%, about 8%, about 9%, about 10%, or any value or range between any two of these values (including endpoints) as compared to milk production in dairy cows that do not ingest the ruminant feed product according to the methods described herein. In particular examples, the milk fat content may increase by an amount of about 10% to about 15%, including about 10%, about 11%, about 12%, about 13%, about 14%, about 15%, or any value or range between any two of these values (including endpoints) as compared to the fat content of milk produced by dairy cows that do not ingest the ruminant feed product according to the methods described herein.

### EXAMPLES

### Example 1 : Feeding a Dairy Cow During a Calving Cycle

A dairy cow is provided with a ruminant feed product prepared to increase the milk fat and the quantity of the milk produced according to a feed program. The ruminant feed product includes a saturated fatty acid component that includes about 90% free palmitic acid, wheat, barley, and sunflower meals. The ruminant feed product also includes Vitamin A and an arginine derivative as nutrient components. The ruminant feed product is in a pellet form. The ruminant has a body weight of about 700 kilograms.

On January 1, 2013, the dairy cow is about 3 weeks before calving (expected calving date of January 22, 2013). The dairy cow is fed about 0.20 grams of the palmitic acid per kilogram of body weight ("grams/kilograms") from a time period starting about 3 weeks before (January 1, 2013) calving until about 1 week before the dairy cow calves (January 15, 2013). Accordingly, from about January 1, 2013 to about January 15, 2013, the dairy cow is fed about 140 grams of the palmitic acid per day.

From about one week before calving (January 16, 2013) until about 1 week after calving (January 29, 2013), the dairy cow is fed an increasing concentration of palmitic acid. The increasing concentration starts at about 0.20 grams/kilograms and ends at about 0.50 grams/kilograms over the time period of about two weeks spanning from January 16, 2013 until January 29, 2013 (13 days). The concentration increase has an increase step of one day such that the concentration of the ruminant feed product increases by (0.50 grams/kilograms - 0.20 grams/kilograms)/13 days ≈ 0.023 grams/kilograms per day. Accordingly, on January 16, 2013, the dairy cow is fed about 140 grams of the ruminant feed product, increasing by about 0.023 grams/kilograms per day or about 16.1 grams per day, to about 350 grams of the ruminant feed product on January 29, 2013.

After January 29, 2013, about 1 week after calving, the dairy cow is in a post-calving or non-calving state and the ruminant is fed based on the milk yield of the ruminant. The concentration of palmitic acid fed to the dairy cow is about 10 grams per kilogram of milk yield per day. On January 30, 2013, the dairy cow has a milk yield of about 40 kilograms of milk per day and, accordingly, is fed about 400 grams of palmitic acid.

As a result of being fed according to the feed program, the dairy cow produces about 10% more milk containing about 10% more milk fat content than when on a diet that did not consist of the ruminant feed product.

### Example 2: Dairy Cow Feeding System

An automated cattle feeding system (the "system") may be configured to feed a plurality of dairy cows a ruminant feed product. The system may include a container for each of the dairy cows and a dispenser to dispense an amount of the ruminant feed product into the container. The ruminant feed product may include a saturated fatty acid component that has a palmitic acid compound content of about 95% and a stearic acid compound content of about 5%. The saturated fatty acid component is substantially free of trans fatty acids. The ruminant feed product includes a carbohydrate component of sugar cane, millet, and barley, a nitrogen component of sunflower meals and linseed meals, and a nutrient component of vitamin B and a mineral of calcium.

The system is configured to receive information indicating a body weight, milk yield, and a calving cycle time period associated with each of the dairy cows. The system is also configured to access a feed program for feeding the dairy cows according to the calving cycle time period and one of the body weight or the milk yield. The dispenser deposits an amount of the ruminant feed product into each container based on the feed program.

The feed program is configured such that a dairy cow is fed (1) about 0.30 grams of palmitic acid per kilogram of body weight ("grams/kilograms") per day from about 2 weeks before calving to about 1 week before calving, (2) about 0.30 grams/kilograms per day from about 1 week before calving to about 0.6 grams/kilograms per day at about 2 weeks after calving, increasing about 0.014 grams/kilograms per day, and (3) about 15 grams per kilogram of milk yield per day after about 2 weeks after calving.

A first container is used by a dairy cow having a milk yield of about 35 kilograms per day that is about 5 weeks after calving. The system dispenses about (35)(15) = 525 grams of the palmitic acid at the first container. A second container is used by a dairy cow having a body weight of about 800 kilograms that is about 2 weeks before calving. The system dispenses about (0.3)(800) = 240 grams of palmitic acid at the second container.

Consumption of the ruminant feed product by the dairy cows results in a daily milk yield increase of about 7% and a milk fat content increase of about 8% compared to dairy cows that do not ingest the palmitic acid ruminant feed product pellets according to the feed program.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity.

It will be understood by those within the art that, in general, terms used herein, and especially in the appended claims (for example, bodies of the appended claims) are generally intended as "open" terms (for example, the term "including" should be interpreted as "including but not limited to", the term "having" should be interpreted as "having at least", the term "includes" should be interpreted as "includes but is not limited to", et cetera). While various compositions, methods, and devices are described in terms of "comprising" various components or steps (interpreted as meaning "including, but not limited to"), the compositions, methods, and devices can also "consist essentially of' or "consist of" the various components and steps, and such terminology should be interpreted as defining essentially closed-member groups. It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to examples containing only one such recitation, even when the same claim includes the introductory phrases "one or more" or "at least one" and indefinite articles such as "a" or "an" (for example, "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (for example, the bare recitation of "two recitations", without other modifiers, means at least two recitations, or two or more recitations). Furthermore, in those instances where a convention analogous to "at least one of A, B, and C, et cetera" is used, in general such a construction is intended in the sense one having skill in the art would understand the convention (for example, "a system having at least one of A, B, and C" would include but not be limited to systems that have A alone, B alone, C alone, A and B together, A and C together, B and C together, and/or A, B, and C together, et cetera). In those instances where a convention analogous to "at least one of A, B, or C, et cetera" is used, in general such a construction is intended in the sense one having skill in the art would understand the convention (for example, " a system having at least one of A, B, or C" would include but not be limited to systems that have A alone, B alone, C alone, A and B together, A and C together, B and C together, and/or A, B, and C together, et cetera). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

As will be understood by one skilled in the art, for any and all purposes, such as in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, et cetera As a non-limiting example, each range discussed herein can be readily broken down into a lower third, middle third and upper third, et cetera As will also be understood by one skilled in the art all language such as "up to," "at least," and the like include the number recited and refer to ranges which can be subsequently broken down into subranges as discussed above. Finally, as will be understood by one skilled in the art, a range includes each individual member. Thus, for example, a group having 1-3 cells refers to groups having 1, 2, or 3 cells. Similarly, a group having 1-5 cells refers to groups having 1, 2, 3, 4, or 5 cells, and so forth.

## Claims

1. A method for feeding a ruminant, the method comprising:
determining a body weight of the ruminant and a milk yield of the ruminant; and
providing a ruminant feed product to the ruminant for ingestion according to a feed program;
wherein the ruminant feed product comprises at least one fatty acid component comprising at least 70% of a palmitic acid compound by weight;
wherein the feed program comprises:
feeding the ruminant feed product to the ruminant at a first level during a first time period, wherein the first level does not exceed 0.04 grams of the fatty acid component per kilogram of the body weight, wherein the first time period comprises a far-off dry cow period in a range of six to three weeks before calving,
feeding the ruminant feed product to the ruminant at a second level during a second time period, wherein the second level comprises at least 0.04 grams of the fatty acid component per kilogram of the body weight, wherein the second time period comprises a close-up dry cow period in a range of three weeks before calving to calving, and
feeding the ruminant feed product to the ruminant at a third level during a third time period, wherein the third level comprises at least 0.4 grams of the fatty acid component per kilogram of the body weight, wherein the third time period is from calving to a far-off dry cow period.

2. The method of claim 1, wherein the first level comprises a ruminant feed dry matter from 1.5% to 2.5% of the body weight.

3. The method of claim 1, wherein the first level does not exceed 0.02 grams of the fatty acid component per kilogram of the body weight.

4. The method of claim 1, wherein the second time period comprises a third week before calving, a second week before calving, and a first week before calving.

5. The method of claim 4, wherein the second level comprises a first concentration during the third week before calving, a second concentration during the second week before calving, and a third concentration during the first week before calving, wherein the first concentration comprises from 0.04 grams to 0.08 grams of the fatty acid component per kilogram of the body weight, wherein the second concentration comprises from 0.08 to 0.16 grams of the fatty acid component per kilogram of the body weight, and wherein the third concentration comprises from 0.14 grams to 0.22 grams of the fatty acid component per kilogram of the body weight.

6. The method of claim 1, wherein the third time period comprises one or more of a fresh cow period, a peak lactation period, a constant phase of lactation period, and a late lactation period, wherein the third level comprises at least 0.4 grams of the fatty acid component per kilogram of the body weight during the fresh cow period, at least 8 grams of the fatty acid component per kilogram of the milk yield during the peak lactation period, 10 grams of the fatty acid component per kilogram of the milk yield during the constant phase of lactation, at least 8 grams of the fatty acid component per kilogram of the milk yield during the late lactation period.

7. The method of claim 1, wherein the third level comprises from 0.4 grams to 0.8 grams of the fatty acid component per kilogram of the body weight during the first week after calving.

8. The method of claim 1, wherein the third level comprises 10 grams of the fatty acid component per kilogram of the milk yield after the first week after calving.

9. The method of claim 1, wherein the at least one saturated fatty acid component comprises 70% to 100% of the palmitic acid compound by weight.

10. The method of claim 1, wherein feeding the ruminant according to the feeding program results in an increase in fat content of milk produced by the ruminant of 10% relative to a substantially similar ruminant not fed according to the feeding program.

## Patentansprüche

1. Verfahren zum Füttern eines Wiederkäuers, wobei das Verfahren umfasst:
Bestimmen eines Körpergewichts des Wiederkäuers und einer Milchproduktion des Wiederkäuers; und
Versorgen des Wiederkäuers mit einem Wiederkäuerfutterprodukt zur Aufnahme gemäß einem Futterprogramm;
wobei das Wiederkäuerfutterprodukt mindestens eine Fettsäurekomponente umfasst, die mindestens 70 Gew.-% einer Palmitinsäureverbindung umfasst;
wobei das Futterprogramm umfasst:
Füttern des Wiederkäuers mit dem Wiederkäuerfutterprodukt auf einer ersten Stufe während einer ersten Zeitperiode, wobei die erste Stufe 0,04 Gramm der Fettsäurekomponente pro Kilogramm des Körpergewichts nicht überschreitet, wobei die erste Zeitperiode eine frühe Trockenstehperiode der Kuh in einem Bereich von sechs bis drei Wochen vor einer Kalbung umfasst,
Füttern des Wiederkäuers mit dem Wiederkäuerfutterprodukt auf einer zweiten Stufe während einer zweiten Zeitperiode, wobei die zweite Stufe mindestens 0,04 Gramm der Fettsäurekomponente pro Kilogramm des Körpergewichts umfasst, wobei die zweite Zeitperiode eine späte Trockenstehperiode der Kuh in einem Bereich von drei Wochen vor der Kalbung bis zur Kalbung umfasst, und
Füttern des Wiederkäuers mit dem Wiederkäuerfutterprodukt auf einer dritten Stufe während einer dritten Zeitperiode, wobei die dritte Stufe mindestens 0,4 Gramm der Fettsäurekomponente pro Kilogramm des Körpergewichts umfasst, wobei die dritte Zeitperiode von der Kalbung zu einer frühen Trockenstehperiode der Kuh reicht.

2. Verfahren nach Anspruch 1, wobei die erste Stufe eine Wiederkäuerfutter-Trockensubstanz von 1,5 % bis 2,5 % des Körpergewichts umfasst.

3. Verfahren nach Anspruch 1, wobei die erste Stufe 0,02 Gramm der Fettsäurekomponente pro Kilogramm des Körpergewichts nicht überschreitet.

4. Verfahren nach Anspruch 1, wobei die zweite Zeitperiode eine dritte Woche vor der Kalbung, eine zweite Woche vor der Kalbung und eine erste Woche vor der Kalbung umfasst.

5. Verfahren nach Anspruch 4, wobei die zweite Stufe eine erste Konzentration während der dritten Woche vor der Kalbung, eine zweite Konzentration während der zweiten Woche vor der Kalbung und eine dritte Konzentration während der ersten Woche vor der Kalbung umfasst, wobei die erste Konzentration von 0,04 Gramm bis 0,08 Gramm der Fettsäurekomponente pro Kilogramm des Körpergewichts umfasst, wobei die zweite Konzentration von 0,08 bis 0,16 Gramm der Fettsäurekomponente pro Kilogramm des Körpergewichts umfasst, und wobei die dritte Konzentration von 0,14 Gramm bis 0,22 Gramm der Fettsäurekomponente pro Kilogramm des Körpergewichts umfasst.

6. Verfahren nach Anspruch 1, wobei die dritte Zeitperiode eine oder mehrere aus einer Frischkalberperiode, einer Laktationsspitzenperiode, einer konstanten Phase einer Laktationsperiode und einer späten Laktationsperiode umfasst, wobei die dritte Stufe mindestens 0,4 Gramm der Fettsäurekomponente pro Kilogramm des Körpergewichts während der Frischkalberperiode, mindestens 8 Gramm der Fettsäurekomponente pro Kilogramm der Milchproduktion während der Laktationsspitzenperiode, 10 Gramm der Fettsäurekomponente pro Kilogramm der Milchproduktion während der konstanten Phase der Laktation, mindestens 8 Gramm der Fettsäurekomponente pro Kilogramm der Milchproduktion während der späten Laktationsperiode umfasst.

7. Verfahren nach Anspruch 1, wobei die dritte Stufe von 0,4 Gramm bis 0,8 Gramm der Fettsäurekomponente pro Kilogramm des Körpergewichts während der ersten Woche nach der Kalbung umfasst.

8. Verfahren nach Anspruch 1, wobei die dritte Stufe 10 Gramm der Fettsäurekomponente pro Kilogramm der Milchproduktion nach der ersten Woche nach der Kalbung umfasst.

9. Verfahren nach Anspruch 1, wobei die mindestens eine gesättigte Fettsäurekomponente 70 Gew.-% bis 100 Gew.-% der Palmitinsäureverbindung umfasst.

10. Verfahren nach Anspruch 1, wobei das Füttern des Wiederkäuers gemäß dem Fütterungsprogramm zu einer Erhöhung des Fettgehalts von durch den Wiederkäuer produzierte Milch von 10 % in Bezug auf einen im Wesentlichen ähnlichen Wiederkäuer, der nicht gemäß dem Fütterungsprogramm gefüttert wurde, führt.

## Revendications

1. Procédé d'alimentation d'un ruminant, le procédé comprenant :
la détermination d'un poids corporel du ruminant et d'une production de lait du ruminant ; et
la fourniture d'un produit alimentaire pour ruminants au ruminant pour ingestion selon un programme d'alimentation ;
dans lequel le produit alimentaire pour ruminants comprend au moins un composant d'acide gras comprenant au moins 70 % d'un composé d'acide palmitique en poids ;
dans lequel le programme d'alimentation comprend :
le nourrissage du ruminant avec le produit alimentaire pour ruminants à un premier niveau pendant une première période, le premier niveau ne dépassant pas 0,04 gramme du composant d'acide gras par kilogramme du poids corporel, la première période comprenant une période de tarissement lointaine allant de six à trois semaines avant le vêlage,
le nourrissage du ruminant avec le produit alimentaire pour ruminants à un deuxième niveau pendant une deuxième période, le deuxième niveau comprenant au moins 0,04 gramme du composant d'acide gras par kilogramme du poids corporel, la deuxième période comprenant une période de tarissement proche allant de trois semaines avant le vêlage au vêlage, et
le nourrissage du ruminant avec le produit alimentaire pour ruminants à un troisième niveau pendant une troisième période, le troisième niveau comprenant au moins 0,4 gramme du composant d'acide gras par kilogramme du poids corporel, la troisième période allant du vêlage à une période de tarissement lointaine.

2. Procédé selon la revendication 1, dans lequel le premier niveau comprend un taux de matière sèche de l'aliment pour ruminants de 1,5 % à 2,5 % du poids corporel.

3. Procédé selon la revendication 1, dans lequel le premier niveau ne dépasse pas 0,02 gramme du composant d'acide gras par kilogramme du poids corporel.

4. Procédé selon la revendication 1, dans lequel la deuxième période comprend une troisième semaine avant le vêlage, une deuxième semaine avant le vêlage et une première semaine avant le vêlage.

5. Procédé selon la revendication 4, dans lequel le deuxième niveau comprend une première concentration pendant la troisième semaine avant le vêlage, une deuxième concentration pendant la deuxième semaine avant le vêlage et une troisième concentration pendant la première semaine avant le vêlage, la première concentration comprenant de 0,04 gramme à 0,08 gramme du composant d'acide gras par kilogramme du poids corporel, la deuxième concentration comprenant de 0,08 gramme à 0,16 gramme du composant d'acide gras par kilogramme du poids corporel, et la troisième concentration comprenant de 0,14 gramme à 0,22 gramme du composant d'acide gras par kilogramme du poids corporel.

6. Procédé selon la revendication 1, dans lequel la troisième période comprend une ou plusieurs d'une période de vache fraîche, d'une période de lactation maximale, d'une période de phase de lactation constante et d'une période de lactation tardive, le troisième niveau comprenant au moins 0,4 gramme du composant d'acide gras par kilogramme du poids corporel pendant la période de vache fraîche, au moins 8 grammes du composant d'acide gras par kilogramme de la production de lait pendant la période de lactation maximale, 10 grammes du composant d'acide gras par kilogramme de la production de lait pendant la phase de lactation constante, au moins 8 grammes du composant d'acide gras par kilogramme de la production de lait pendant la période de lactation tardive.

7. Procédé selon la revendication 1, dans lequel le troisième niveau comprend de 0,4 gramme à 0,8 gramme du composant d'acide gras par kilogramme du poids corporel pendant la première semaine après le vêlage.

8. Procédé selon la revendication 1, dans lequel le troisième niveau comprend 10 grammes du composant d'acide gras par kilogramme de la production de lait après la première semaine après le vêlage.

9. Procédé selon la revendication 1, dans lequel l'au moins un composant d'acide gras saturé comprend 70 % à 100 % du composé d'acide palmitique en poids.

10. Procédé selon la revendication 1, dans lequel l'alimentation du ruminant selon le programme d'alimentation entraîne une augmentation de la teneur en matière grasse du lait produit par le ruminant de 10 % par rapport à un ruminant sensiblement similaire non alimenté selon le programme d'alimentation.
